# EUROPEAN PATENT APPLICATION

(11) **EP 4 647 101 A1**
(43) Date of publication of application: **12.11.2025**
(21) Application number: 24784594.4
(22) Date of filing: 09.02.2024
(51) Int. Cl.: A61M 16/10

(54) **OXYGEN CONCENTRATION DEVICE**

(30) Priority: 03.04.2023 JP 2023060140
(71) Applicant: Daikin Industries, Ltd., Osaka-shi, Osaka 530-0001 (JP)
(72) Inventor: NAGAOKA, Namiharu, Osaka-shi, Osaka 530-0001 (JP); KONDO, Keita, Osaka-shi, Osaka 530-0001 (JP); MOCHIZUKI, Osamu, Osaka-shi, Osaka 530-0001 (JP); YAMAMOTO, Kenta, Osaka-shi, Osaka 530-0001 (JP); NAKAMURA, Hirofumi, Osaka-shi, Osaka 530-0001 (JP); OSHIKAWA, Takero, Osaka-shi, Osaka 530-0001 (JP)
(74) Representative: Goddar, Heinz J.
(86) International application number: PCT/JP2024/004486
(87) International publication number: WO 2024/209791

(57) **Abstract**

An oxygen concentrator M includes: an adsorption cylinder 20 having an adsorbent that selectively adsorbs nitrogen from raw air and generates high-concentration oxygen; and a humidifying device 30 that humidifies the generated high-concentration oxygen. The humidifying device 30 includes a winding body 32 composed of a humidifying tube 31 that supplies moisture present outside to the high-concentration oxygen flowing through an interior, the winding body 32 being formed by winding the single humidifying tube 31 around a virtual axis P.

## Description

### TECHNICAL FIELD

The present disclosure relates to an oxygen concentrator.

### BACKGROUND ART

An oxygen concentrator that generates air containing a higher concentration of oxygen than the oxygen concentration in air (hereinafter referred to as high-concentration oxygen) and supplies the generated air to a user is known. The oxygen concentrator includes a humidifying device that humidifies the generated high-concentration oxygen. As the humidifying device, a type that has a water replenishment tank and a type that uses a humidifying tube are known. The humidifying device of the type that uses a humidifying tube can eliminate the need for water replenishment. An example of the oxygen concentrator including the humidifying device of the type that uses a humidifying tube is disclosed in Patent Literature 1.

The humidifying device disclosed in Patent Literature 1 is used in a state where a plurality of humidifying tubes (hollow fibers that form a moisture-permeable membrane) are bundled and extended linearly. The humidifying device passes high-concentration oxygen (oxygen-enriched air) through the humidifying tube and also causes raw air to flow into a case (moisture-permeable membrane module body) that houses a bundle of the humidifying tube, thereby providing moisture contained in the raw air to the high-concentration oxygen.

### CITATION LIST

### [PATENT LITERATURE]

PATENT LITERATURE 1: Japanese Laid-Open Patent Publication No. 2007-282652

### SUMMARY OF THE INVENTION

### [TECHNICAL PROBLEM]

When a plurality of humidifying tubes are used in a bundle, there will be areas where adjacent humidifying tubes overlap (contact) each other. In the above mentioned areas, the surface of the humidifying tube is unlikely to come into contact with air. Such a humidifying device cannot effectively utilize the entire surface of the humidifying tube for acquiring moisture. Therefore, conventional oxygen concentrators, when including a humidifying device of the type that uses a humidifying tube, sometimes have an insufficient amount of humidification for high-concentration oxygen.

An object of the present disclosure is to increase the amount of humidification for high-concentration oxygen in an oxygen concentrator including the humidifying device of the type that uses a humidifying tube, as compared to the conventional oxygen concentrators.

### [SOLUTION TO PROBLEM]

(1) An oxygen concentrator of the present disclosure includes: an adsorption cylinder having an adsorbent that selectively adsorbs nitrogen from raw air and generates high-concentration oxygen; and a humidifying device that humidifies the generated high-concentration oxygen. The humidifying device includes a winding body composed of a humidifying tube that supplies moisture present outside to the high-concentration oxygen flowing through an interior, the winding body being formed by winding the single humidifying tube around a virtual axis.
   The humidifying device of the oxygen concentrator of the present disclosure includes the winding body formed by winding the humidifying tube. The winding body of the humidifying tube has less surface overlap between the humidifying tubes, as compared to the case where the humidifying tubes are used in a bundle. Therefore, the humidifying device included in the oxygen concentrator of the present disclosure has a larger surface area of the humidifying tube that can be effectively utilized for acquiring moisture, as compared to the case where the same total length of the humidifying tube is used in a bundle. Therefore, the humidifying device with such a configuration can enhance the humidification capacity for the high-concentration oxygen, as compared to conventional humidifying devices. This allows the oxygen concentrator including the humidifying device of the type that uses the humidifying tube to increase the amount of humidification for the high-concentration oxygen, as compared to conventional oxygen concentrators.
(2) In the oxygen concentrator according to (1) described above of the present disclosure, preferably, the winding body has a form that is wound in a spiral shape around the virtual axis.
   The humidifying device with the above configuration can effectively utilize the surface area of the humidifying tube for acquiring moisture. This allows the humidification capacity of the humidifying device for the high-concentration oxygen to be enhanced.
(3) In the oxygen concentrator according to (1) or (2) described above of the present disclosure, preferably, the winding body has a form that is wound in a helical shape around the virtual axis.
   The humidifying device with the above configuration can effectively utilize the surface area of the humidifying tube for acquiring moisture. This allows the humidification capacity of the humidifying device for the high-concentration oxygen to be enhanced.
(4) In the oxygen concentrator according to any one of aspects (1) to (3) described above of the present disclosure, preferably, the humidifying device further includes a holder that holds the winding body, and the holder holds the humidifying tube in a state where the adjacent humidifying tubes are spaced apart from each other.
   The humidifying device with the above configuration can make maximum effective use of the surface area of the humidifying tube for acquiring moisture. This allows the humidification capacity of the humidifying device for the high-concentration oxygen to be enhanced more.
(5) In the oxygen concentrator according to any one of aspects (1) to (4) described above of the present disclosure, preferably, the winding body includes a first winding body and a second winding body, and the first winding body and the second winding body are disposed coaxially adjacent to each other in a direction of the virtual axis.
   The oxygen concentrator with the above configuration, which includes the two humidifying tubes, can increase the surface area of the humidifying tubes that can be utilized for acquiring moisture in the humidifying device.
(6) The oxygen concentrator according to any one of aspects (1) to (5) described above of the present disclosure preferably further includes: an air pump that supplies the raw air to the adsorption cylinder; a cooling fan that blows cooling air to the air pump; and a housing that includes an air supply port that takes in the cooling air from outside and constitutes a flow path for the cooling air that flows from the air supply port to the cooling fan, and the humidifying device is preferably disposed at a position away from a flow of the cooling air in the flow path.
   The oxygen concentrator of the present disclosure can enhance the flexibility of the placement of the humidifying device by disposing the humidifying device at a position away from the flow of the cooling air generated by the cooling fan. This allows the space efficiency of the oxygen concentrator including the humidifying device to be enhanced, enabling further downsizing of the oxygen concentrator.
(7) In the oxygen concentrator according to (6) described above of the present disclosure, preferably, the housing includes a cooling fan housing that houses the cooling fan, and the humidifying device is disposed outside the cooling fan housing.

The oxygen concentrator of the present disclosure can further enhance the flexibility of the placement of the humidifying device by disposing the humidifying device outside the cooling fan housing. This allows the space efficiency of the oxygen concentrator including the humidifying device to be enhanced, enabling further downsizing of the oxygen concentrator.

### BRIEF DESCRIPTION OF DRAWINGS

[FIG. 1] FIG. 1 is an explanatory diagram of an oxygen concentrator according to one embodiment of the present disclosure.
[FIG. 2] FIG. 2 is an explanatory diagram of placement of a humidifying device and air flow in the oxygen concentrator.
[FIG. 3] FIG. 3 is a schematic perspective view showing the humidifying device.
[FIG. 4] FIG. 4 is an exploded view showing the humidifying device.
[FIG. 5A] FIG. 5A is a schematic cross-sectional view of a humidifying tube.
[FIG. 5B] FIG. 5B is a schematic cross-sectional view showing placement of the humidifying tube in a winding body.
[FIG. 6A] FIG. 6A is a schematic cross-sectional view of the winding body that has a helical shape.
[FIG. 6B] FIG. 6B is a schematic cross-sectional view of the winding body that has a helical shape and spiral shape.

### DETAILED DESCRIPTION

An oxygen concentrator according to the present disclosure will be described in detail below with reference to the accompanying drawings.

### [About overall configuration of oxygen concentrator of the present disclosure]

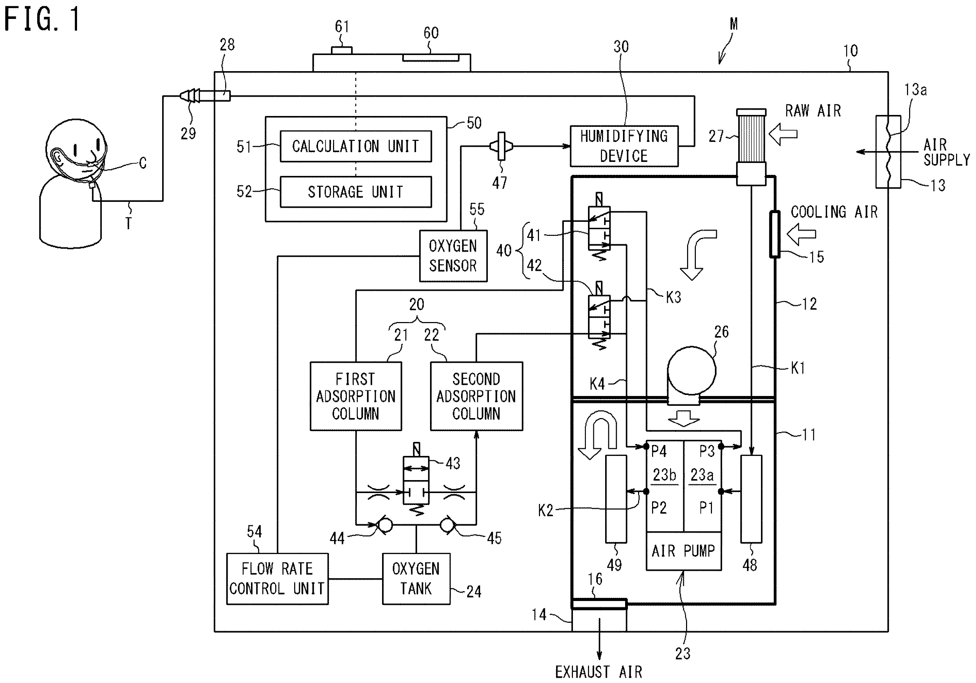

FIG. 1 is an explanatory diagram of the oxygen concentrator according to one embodiment of the present disclosure. The oxygen concentrator of the present disclosure is a device that generates high-concentration oxygen containing oxygen having oxygen concentration higher than oxygen concentration in air and supplies the generated high-concentration oxygen to a user. The oxygen concentrator is used, for example, in home oxygen therapy for providing high-concentration oxygen to a respiratory disease patient or the like who is a user.

An oxygen concentrator M shown in FIG. 1 is one embodiment of the oxygen concentrator of the present disclosure. As shown in FIG. 1, the oxygen concentrator M of the present disclosure includes a casing (housing) 10, an adsorption cylinder 20, and a humidifying device 30. The casing 10 is an outer cover of the oxygen concentrator M and constitutes an outer shell of the oxygen concentrator M. The oxygen concentrator M includes a pump box 11 and a fan box 12 that are built into the casing 10. The pump box 11 is primarily a housing that houses an air pump, and the fan box 12 is primarily a housing that houses a fan.

The adsorption cylinder 20 adsorbs nitrogen from air supplied to the adsorption cylinder 20, generating high-concentration oxygen and low-concentration oxygen. The oxygen concentrator M includes a first adsorption cylinder 21 and a second adsorption cylinder 22 as the adsorption cylinder 20. Note that the low-concentration oxygen is air with a higher nitrogen concentration than normal air (nitrogen-rich gas) and contains oxygen with lower oxygen concentration than the oxygen concentration in the air. The adsorption cylinder 20 includes an adsorbent that selectively adsorbs nitrogen from the air. In this description, the air supplied to the adsorption cylinder 20 to generate high-concentration oxygen is referred to as raw air.

The humidifying device 30 provides moisture to the high-concentration oxygen generated in the adsorption cylinder 20. The humidifying device 30 of the oxygen concentrator M of the present disclosure is of the type that uses a humidifying tube (humidifying tube 31, which will be described later) and does not need to replenish water. The configuration of the humidifying device 30 will be described in detail later.

The oxygen concentrator M further includes an air pump 23 that supplies pressurized raw air (pressurized air) to the first adsorption cylinder 21 and the second adsorption cylinder 22, and an oxygen tank 24 that stores the high-concentration oxygen. The air pump 23 of the present embodiment is a combined pressure/vacuum type fluid machine that can perform both pressurization and suction of gas such as air. The air pump 23 includes a pressurizing unit 23a that functions as a compressor to supply pressurized air, and an exhaust unit 23b that functions as a vacuum pump to suction and exhaust gas such as air. The air pump 23 includes a first port P1, a second port P2, a third port P3, and a fourth port P4. The first port P1 is an inlet port of the pressurizing unit 23a, and the third port P3 is an outlet port of the pressurizing unit 23a. The second port P2 is an outlet port of the exhaust unit 23b, and the fourth port P4 is an inlet port of the exhaust unit 23b.

The oxygen concentrator M includes an air supply route K1 connected to the first port P1 of the pressurizing unit 23a, and an air supply route K3 connecting the third port P3 of the pressurizing unit 23a to the first adsorption cylinder 21 and the second adsorption cylinder 22. The oxygen concentrator M includes an exhaust route K2 connected to the second port P2 of the exhaust unit 23b, and an exhaust route K4 connecting the fourth port P4 of the exhaust unit 23b to the first adsorption cylinder 21 and the second adsorption cylinder 22.

The air pump 23 supplies pressurized air to the first adsorption cylinder 21 and the second adsorption cylinder 22 via the air supply routes K1 and K3 by the pressurizing unit 23a, and decompresses inside the first adsorption cylinder 21 and the second adsorption cylinder 22 via the exhaust routes K2 and K4 by the exhaust unit 23b, thereby desorbing nitrogen from the adsorbent and exhausting low-concentration oxygen (nitrogen-rich gas). Note that in the present embodiment, the combined pressure/vacuum type air pump 23 is used; however, the oxygen concentrator of the present disclosure may also have a configuration in which a compressor that supplies pressurized air and a vacuum pump for decompressing inside the adsorption cylinder are included separately in the device. The oxygen tank 24 stores the high-concentration oxygen generated in the first adsorption cylinder 21 and the second adsorption cylinder 22. The first adsorption cylinder 21, the second adsorption cylinder 22, and the oxygen tank 24 are disposed in the casing 10.

The oxygen concentrator M further includes a switching valve 40. The switching valve 40 switches the flow of pressurized air from the pressurizing unit 23a to the first adsorption cylinder 21 and the second adsorption cylinder 22, and the flow of exhaust air (low-concentration oxygen) from the first adsorption cylinder 21 and the second adsorption cylinder 22 to the exhaust unit 23b. The switching valve 40 in the present embodiment includes a first control valve 41, which is a three-port valve, and a second control valve 42, which is also a three-port valve.

The oxygen concentrator M further includes a control unit 50. The operation of the air pump 23 and the operation of various control valves described later are performed by the control unit 50 disposed inside the device. The control unit 50 includes a storage unit 51 in which a program for operating the oxygen concentrator M is stored, and a calculation unit 52 that issues operation signals for control valves and the like. The control unit 50 is connected to a display unit 60 that displays information such as the operating state of the oxygen concentrator M, and an operation unit 61 that allows the user to operate the oxygen concentrator M.

In the oxygen concentrator M, the pump box 11 houses the air pump 23, an air supply silencer 48, and an exhaust air silencer 49. In the oxygen concentrator M, the fan box 12 houses a cooling fan 26 and the switching valve 40.

### [About air flow]

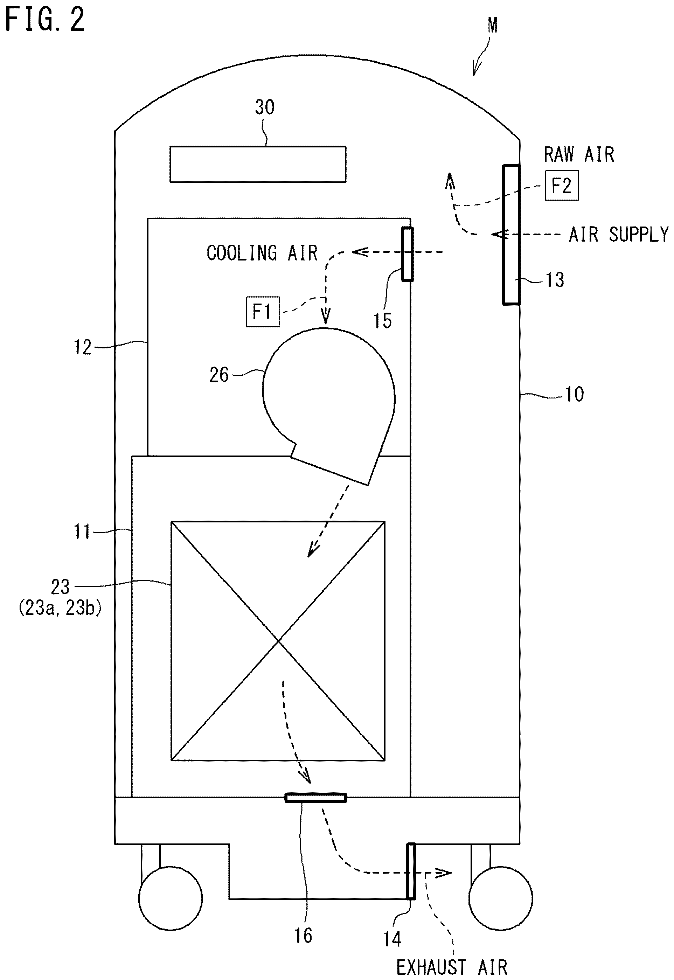

FIG. 2 is an explanatory diagram of placement of the humidifying device and air flow in the oxygen concentrator. Note that in FIG. 2, for the convenience of description, illustrations of the air supply silencer 48 and the exhaust air silencer 49 in the pump box 11, the switching valve 40 in the fan box 12, and the like are omitted. As shown in FIGS. 1 and 2, the casing 10 includes an air supply port 13 for taking in air into the casing 10 and an exhaust port 14 for exhausting air from the casing 10. The air supply port 13 is provided with a dust filter 13a to collect dust and other particles contained in the air. The air taken in from the air supply port 13 includes the raw air and the cooling air for cooling the air pump 23.

The fan box 12 includes an air supply port 15 for taking in the cooling air into the fan box 12. When the cooling fan 26 operates, air is taken into the fan box 12 via the air supply ports 13 and 15. The cooling air that is taken into the fan box 12 is drawn in by the cooling fan 26 and is discharged toward the pump box 11. In this description, the air flow that is taken into the cooling fan 26 through the air supply ports 13 and 15 is referred to as flow F1. The flow F1 is mainly the flow of the cooling air. In the oxygen concentrator M of the present disclosure, the casing 10 and the fan box 12 constitute the flow path of the cooling air flowing from the air supply ports 13 and 15 to the cooling fan 26. The flow F1 flows through the flow path from the air supply port 13 to the cooling fan 26.

The heat generated by the operation of the air pump 23 is cooled by the cooling air blown onto the air pump 23 by the cooling fan 26. The cooling air, after being blown onto the air pump 23, is exhausted from the exhaust port 14 to the outside of the pump box 11.

When the air pump 23 is operating, a portion of the air taken into the casing 10 is drawn in from an air supply filter 27 and is drawn into the pressurizing unit 23a via the air supply route K1 and the air supply silencer 48. In this description, the air flow that is taken into the casing 10 from the air supply port 13 and then flows toward the air supply filter 27 is referred to as flow F2. The flow F2 is mainly the flow of the raw air.

The air supply silencer 48 is disposed in the air supply route K1 for the raw air from the air supply filter 27 to the air pump 23. The air supply silencer 48 reduces noise caused by the air supply and compression in the pressurizing unit 23a of the air pump 23. The exhaust air silencer 49 is disposed in the exhaust route K2 for the low-oxygen air exhausted from the exhaust unit 23b. The exhaust air silencer 49 reduces noise caused by the exhaust air from the exhaust unit 23b of the air pump 23.

The adsorbent that selectively or preferentially adsorbs nitrogen in the pressurized air supplied from the air pump 23 is housed inside the first adsorption cylinder 21 and the second adsorption cylinder 22. As the adsorbent, for example, zeolite or the like can be used.

Various valves to control the flow rate or flow of the fluid such as high-concentration oxygen, that is, a purge valve 43, and check valves 44 and 45 are provided in the flow path downstream of the first adsorption cylinder 21 and the second adsorption cylinder 22 (flow path on the outlet side of the high-concentration oxygen; in FIG. 1, the flow path from the lower part of the first adsorption cylinder 21 and the second adsorption cylinder 22 to an oxygen outlet 28). The oxygen tank 24 is provided downstream of the check valves 44 and 45. The high-concentration oxygen is sent to the oxygen outlet 28 via a flow rate control unit 54 for controlling the flow rate of the high-concentration oxygen and an oxygen sensor 55 for detecting oxygen concentration abnormalities. Note that the flow path that supplies the high-concentration oxygen is appropriately provided with a pressure sensor (not shown) for detecting pressure abnormalities and the like, and a decompression valve (not shown) for adjusting the pressure.

The check valve 44 is disposed in the gas flow path downstream of the first adsorption cylinder 21, and the check valve 45 is disposed in the gas flow path downstream of the second adsorption cylinder 22. The check valves 44 and 45 are both configured such that the high-concentration oxygen exhausted from the first adsorption cylinder 21 and the second adsorption cylinder 22 flows only toward the downstream side. The purge valve 43 is disposed in a gas flow path that connects the gas flow path between the first adsorption cylinder 21 and the check valve 44, and the gas flow path between the second adsorption cylinder 22 and the check valve 45.

The high-concentration oxygen from the check valve 44 and the high-concentration oxygen from the check valve 45 are alternately supplied to the oxygen tank 24 and stored in the oxygen tank 24. A bacteria filter 47 for removing foreign substances from the high-concentration oxygen and the humidifying device 30 for humidifying the generated high-concentration oxygen are disposed downstream of the oxygen tank 24. In the oxygen concentrator M, the oxygen outlet 28 is provided with a cannula joint 29, and the high-concentration oxygen is supplied to a patient via a tube T and a cannula C connected to the cannula joint 29.

The first control valve 41 and the second control valve 42 are both three-port valves that switch between a pressurized state in which pressurized air discharged from the air pump 23 is supplied to the first adsorption cylinder 21 (second adsorption cylinder 22), and a decompressed state in which exhaust gas inside the first adsorption cylinder 21 (second adsorption cylinder 22) is exhausted to the outside by suction. When one of the adsorption cylinders is in the pressurized state, the other adsorption cylinder is in the decompressed state.

### [About the process for generating high-concentration oxygen]

As shown in FIG. 1, the raw air drawn in from the air supply filter 27 into the pressurizing unit 23a via the air supply route K1 and the air supply silencer 48 is pressurized (compressed) by the pressurizing unit 23a. The pressurized raw air is supplied to the first adsorption cylinder 21 and the second adsorption cylinder 22 via the air supply route K3, the first control valve 41, and the second control valve 42. The exhaust gas from the first adsorption cylinder 21 and the second adsorption cylinder 22 is decompressed and drawn in by the exhaust unit 23b via the exhaust route K4, the first control valve 41, and the second control valve 42, and is exhausted to the outside of the casing 10 from an opening 16 and the exhaust port 14 through the exhaust route K2 and the exhaust air silencer 49.

The oxygen concentrator M performs the process of alternately pressurizing and decompressing the inside of the first adsorption cylinder 21 and the second adsorption cylinder 22 by switching the opening and closing states of the first control valve 41, the second control valve 42, and the purge valve 43, thereby generating the high-concentration oxygen. Note that the oxygen concentrator M shown in the present embodiment is a vacuum pressure swing adsorption system (VPSA) type oxygen concentrator in which, while the air compressed by the air pump 23 is supplied to one of the adsorption cylinders, the other adsorption cylinder is decompressed by being drawn in by the air pump 23. However, the oxygen concentrator of the present disclosure is not limited to this type, and can also be a pressure swing adsorption system (PSA) type oxygen concentrator in which, while the air compressed by a compressor is supplied to one adsorption cylinder, the other adsorption cylinder is decompressed by being opened to the atmosphere.

### [About humidifying device]

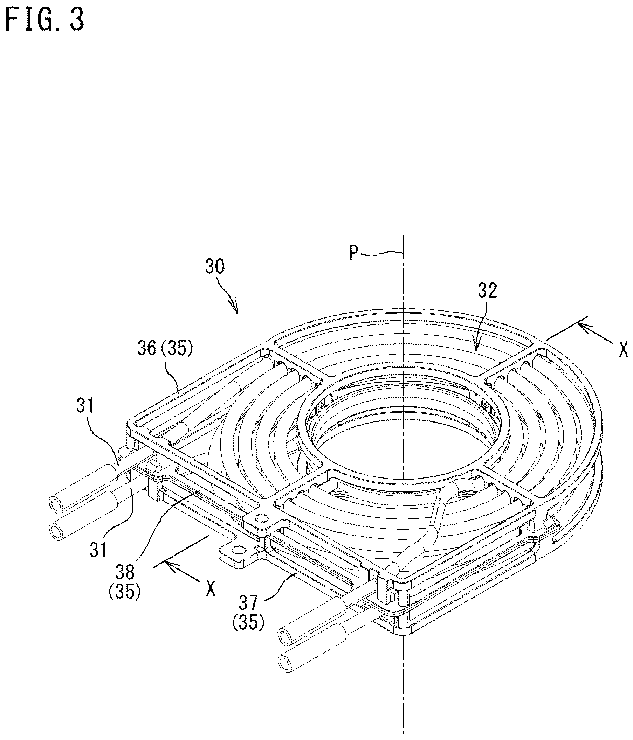

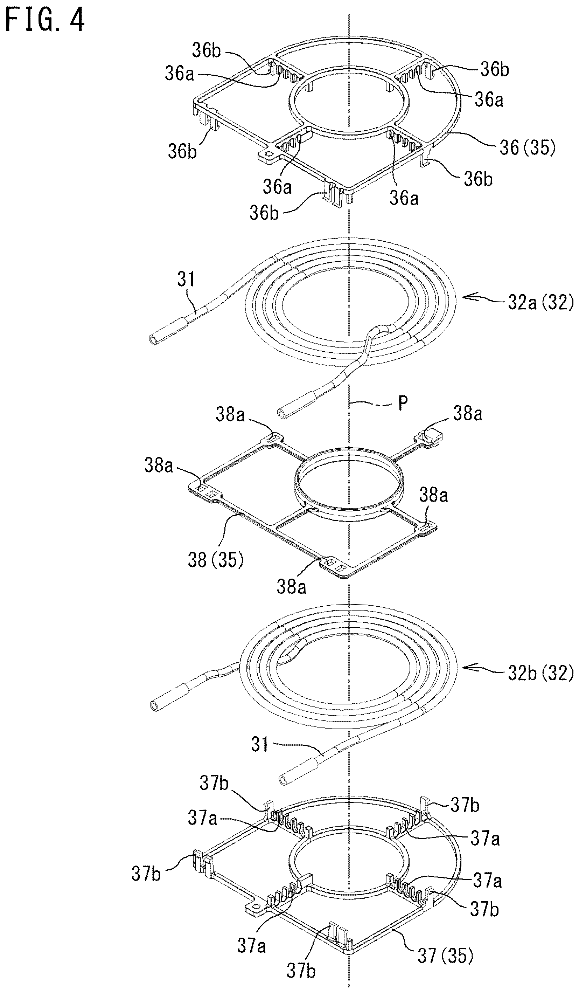

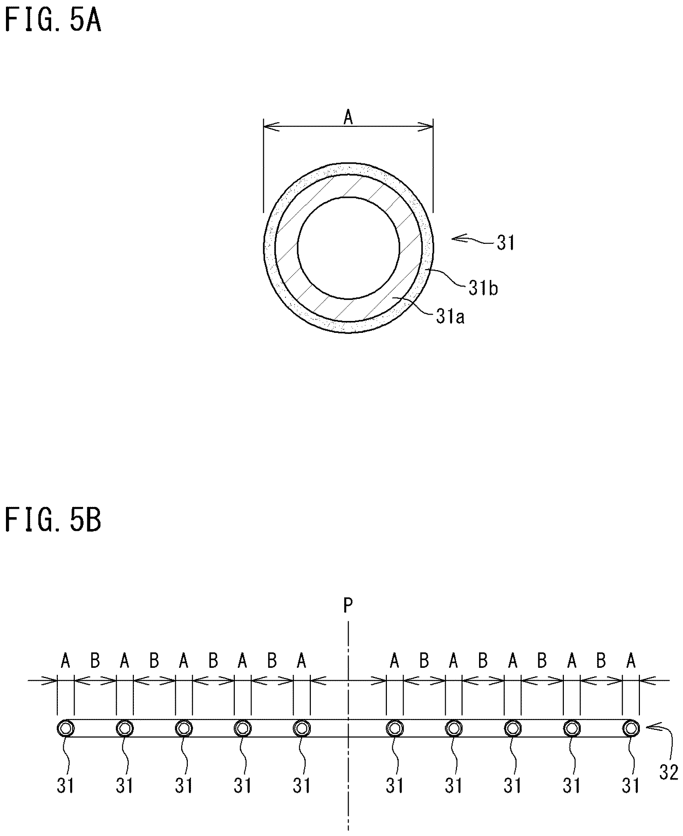

FIG. 3 is a schematic perspective view showing the humidifying device. FIG. 4 is an exploded view showing the humidifying device. FIG. 5A is a schematic cross-sectional view of the humidifying tube. FIG. 5B is a schematic cross-sectional view showing placement of the humidifying tube in a winding body. As shown in FIGS. 3 and 4, the humidifying device 30 includes the humidifying tube 31. The humidifying tube 31 is a hollow fiber composed of a moisture-permeable membrane, acquires moisture from a gas (air) outside the tube, and provides the acquired moisture to the high-concentration oxygen flowing through the inside of the tube. The humidifying device 30 uses the hollow fiber cut to a predetermined length as the humidifying tube 31. The predetermined length is determined based on the amount of moisture (amount of humidification) to be provided to the high-concentration oxygen by the single humidifying tube 31.

As shown in FIG. 5A, the humidifying tube 31 used in the humidifying device 30 of the present embodiment includes a tube body 31a composed of a moisture-permeable membrane and a protective layer 31b that covers the outer peripheral surface of the tube body 31a. The protective layer 31b includes a material that is permeable to air (for example, metal mesh). Therefore, in the humidifying tube 31, the protective layer 31b does not hinder taking in moisture from the surface of the tube body 31a. When the adjacent humidifying tubes 31 come into contact with each other, a space corresponding to the protective layer 31b is secured between the tube bodies 31a at the contact area of the adjacent humidifying tubes 31. Therefore, even in the case where the adjacent humidifying tubes 31 come into contact with each other, the humidifying tube 31 can effectively utilize the surface of the tube body 31a of the contact area for acquiring moisture.

The humidifying device 30 includes a winding body 32 obtained by winding the single humidifying tube 31 around a virtual axis P. The winding body 32 shown in FIG. 3 is Embodiment 1 of the winding body 32. The winding body 32 shown in FIGS. 3 and 4 is configured by winding the single humidifying tube 31 in a "spiral shape" around the virtual axis P. Note that the "spiral shape" as used in this description means a form in which the distance of the humidifying tube 31 from the axis P changes as the humidifying tube 31 is wound around the axis P. When winding the humidifying tube 31 around the axis P from the radially inner side to the outer side, the distance of the humidifying tube 31 from the axis P increases monotonically. When winding the humidifying tube 31 around the axis P from the radially outer side to the inner side, the distance of the humidifying tube 31 from the axis P decreases monotonically.

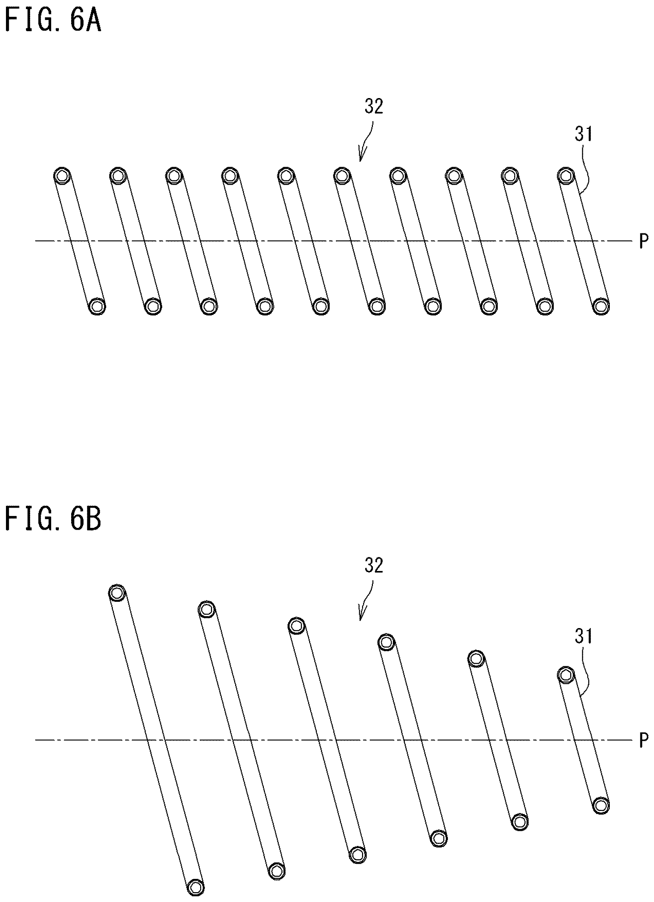

FIG. 6A is a schematic cross-sectional view of the winding body that has a helical shape. FIG. 6B is a schematic cross-sectional view of the winding body that has a helical shape and spiral shape. The winding body 32 that constitutes the humidifying device 30 may be in the form shown in FIGS. 6A and 6B. The winding body 32 shown in FIG. 6A is Embodiment 2 of the winding body 32. The winding body 32 shown in FIG. 6A is configured by winding the single humidifying tube 31 in the "helical shape" around the virtual axis P. Note that the "helical shape" as used in this description means a form in which the position of the humidifying tube 31 in the axial direction of the axis P changes as the humidifying tube 31 is wound around the axis P.

The winding body 32 shown in FIG. 6B is Embodiment 3 of the winding body 32. The winding body 32 that constitutes the humidifying device 30 may be configured by winding the single humidifying tube 31 in a "spiral shape" and "helical shape" around the virtual axis P.

As shown in FIG. 4, the humidifying device 30 of the present embodiment includes two winding bodies 32 (two humidifying tubes 31). In the following description, among the two winding bodies 32 of the humidifying device 30, one is referred to as a first winding body 32a, whereas the other is referred to as a second winding body 32b. In the humidifying device 30 of the present embodiment, the first winding body 32a and the second winding body 32b are disposed coaxially adjacent to each other in the axial direction of the axis P (with their axes P aligned). Such a humidifying device 30 increases the surface area of the humidifying tube 31 that can be utilized for acquiring moisture, while compactly disposing the two winding bodies 32. The humidifying device 30 suppresses the increase in the external dimension of the humidifying device 30 while increasing the surface area of the humidifying tube 31.

In the humidifying device 30 of the present embodiment, the first winding body 32a and the second winding body 32b are connected in parallel in the flow path of the high-concentration oxygen. Therefore, the humidifying device 30 of the present embodiment can enhance the humidification capacity without reducing the supply capacity of the high-concentration oxygen. Note that in the humidifying device 30 of the present embodiment, the first winding body 32a and the second winding body 32b may be connected in series in the flow path of the high-concentration oxygen.

The humidifying device 30 further includes a holder 35. The holder 35 holds the winding body 32 composed of the humidifying tube 31. In the humidifying device 30 of the present embodiment, the winding body 32 is configured by being held by the holder 35 in a state where the humidifying tube 31 is wound.

FIG. 5B shows the cross section of the humidifying tube 31 along the X-X line of FIG. 3. In the winding body 32 held by the holder 35, as shown in FIG. 5B, a gap B is secured between the adjacent humidifying tubes 31. By securing the gap B, the winding body 32 almost eliminates any portions of the surface of the humidifying tube 31 that do not come into contact with air, thereby making maximum effective use of the surface area of the humidifying tube 31 for acquiring moisture. This allows the humidification capacity of the humidifying device 30 for the high-concentration oxygen to be further enhanced. Note that the gap B of the winding body 32 may be "0". If the gap B is set to "0", the winding body 32 can be configured more compactly.

As shown in FIGS. 3 and 4, the holder 35 includes a first holder 36, a second holder 37, and a separator 38. The first holder 36, the second holder 37, and the separator 38 are all resin-made frame bodies. The first holder 36 holds the first winding body 32a. The second holder 37 holds the second winding body 32b.

The first holder 36 includes a plurality of groove portions 36a and a plurality of claw portions 36b. Each of the groove portions 36a is a concave portion that can fit and hold the humidifying tube 31, having a groove width that substantially matches the outer diameter A of the humidifying tube 31 (see FIG. 5A). The plurality of groove portions 36a is arranged along a path traced by the axis of the humidifying tube 31 configured as the winding body 32. Therefore, by fitting the humidifying tube 31 into the plurality of groove portions 36a in predetermined order, the first winding body 32a wound around the axis P is formed. Each of the claw portions 36b has a hook-like form that can be hooked into a hole formed in the separator 38 (locking portion 38a, which will be described later).

The second holder 37 includes a plurality of groove portions 37a and a plurality of claw portions 37b. Each of the groove portions 37a is a concave portion that can fit and hold the humidifying tube 31, having a groove width that substantially matches the outer diameter A of the humidifying tube 31 (see FIG. 5A). The plurality of groove portions 37a is arranged along a path traced by the axis of the humidifying tube 31 configured as the winding body 32. Therefore, by fitting the humidifying tube 31 into the plurality of groove portions 37a in predetermined order, the second winding body 32b wound around the axis P is formed. Each of the claw portions 37b has a hook-like form that can be hooked into a hole formed in the separator 38 (locking portion 38a, which will be described later). For the convenience of description, the second holder 37 is denoted with a different sign from the first holder 36; however, the first holder 36 and the second holder 37 are members that have the same shape and dimensions. The first holder 36 and the second holder 37 are used in a state where the front and back are reversed.

The separator 38 is disposed between the first holder 36 and the second holder 37 in the direction of the axis P. The separator 38 serves the role of preventing contact between the first winding body 32a held by the first holder 36 and the second winding body 32b held by the second holder 37. The separator 38 also has the role of a member that couples the first holder 36 and the second holder 37. The separator 38 includes a plurality of the locking portions 38a. Each of the locking portions 38a includes one or two holes. The locking portion 38a can lock each of the claw portions 36b and 37b that are inserted into the holes.

The holder 35 is integrally configured by locking the claw portion 36b with the locking portion 38a in a state in which the first holder 36 is placed adjacent to a first surface side of the separator 38, and by locking the claw portion 37b with the locking portion 38a in a state in which the second holder 37 is placed adjacent to a second surface side of the separator 38. By using the holder 35 with such a configuration, the humidifying device 30 can dispose the first winding body 32a and the second winding body 32b coaxially adjacent to each other in the direction of the virtual axis P. The oxygen concentrator M that includes such two winding bodies 32 (two humidifying tubes 31) can increase the surface area of the humidifying tubes 31 that can be utilized for acquiring moisture.

The holder 35 of the present embodiment includes the first holder 36 and the second holder 37 constructed from the same member. Therefore, the oxygen concentrator M of the present disclosure can configure the holder 35 with a small number of components, and can suppress the manufacturing cost of the holder 35. Note that the holder 35 may be configured such that the claw portion 36b and the claw portion 37b are directly coupled, allowing the first holder 36 and the second holder 37 to be coupled without interposing the separator 38. In this case, the holder 35 may omit the separator 38.

### [About placement of the humidifying device]

The humidifying device 30 included in the oxygen concentrator M of the present disclosure can suppress overlap (contact) between adjacent humidifying tubes 31, as compared to a case in which a plurality of humidifying tubes 31 is used in a bundle. This makes it possible to effectively utilize the entire surface of the humidifying tube 31 for acquiring moisture. Therefore, the humidifying device 30 of the present disclosure can provide a larger amount of moisture to the high-concentration oxygen, as compared to a conventional humidifying device using humidifying tubes of the same total length (can increase the amount of humidification). Therefore, the humidifying device 30 can ensure the desired humidification capacity without having to be disposed at a selected position in an air flow (for example, flow F1 of the cooling air), unlike conventional humidifying devices.

As shown in FIGS. 1 and 2, in the oxygen concentrator M, the humidifying device 30 is disposed outside the fan box 12. The humidifying device 30 is disposed at a position away from the flow F1 of the cooling air. By disposing the humidifying device 30 at a position away from the flow F1 of the cooling air, the oxygen concentrator M enhances the flexibility of the placement of the humidifying device 30.

In the conventional oxygen concentrator, in order to ensure humidification capacity by disposing the humidifying device at a selected position in an air flow (for example, flow F1 of the cooling air), the humidifying device is disposed inside the housing that houses the cooling fan. Therefore, in the conventional oxygen concentrator, it is unavoidable to dispose the humidifying device in a limited space within the housing, inevitably leading to a larger housing that houses a fan, thus becoming a factor that hinders downsizing of the oxygen concentrator.

The humidifying device 30 of the present embodiment, which has a higher humidification capacity than conventional humidifying devices, can secure the desired humidification capacity without disposing the humidifying device 30 in the flow F1 of the cooling air.

The oxygen concentrator M of the present disclosure eliminates the need to enlarge the fan box 12 by disposing the humidifying device 30 outside the fan box 12 (at a position away from the flow F1 of the cooling air), thereby enabling downsizing of the oxygen concentrator M.

Furthermore, in the oxygen concentrator M of the present disclosure, the humidifying device 30 is disposed in the vicinity of the air supply filter 27 above the fan box 12 (see FIG. 1). This position is in the vicinity of the flow F2 of the raw air. The oxygen concentrator M of the present disclosure promotes the exchange of air present around the humidifying device 30 due to the flow F2 of the raw air shown in FIG. 2. Therefore, even when the humidifying device 30 is disposed at a position away from the flow F1 of the cooling air, it is possible to secure the desired humidification capacity through the synergistic effect of the improvement in the humidification capacity of the humidifying device 30 itself.

### [Functional effects of embodiment]

(1) The oxygen concentrator M of the above embodiment includes the adsorption cylinder 20 that has an adsorbent that selectively adsorbs nitrogen from the raw air to generate high-concentration oxygen, and the humidifying device 30 that humidifies the generated high-concentration oxygen. In the oxygen concentrator M, the humidifying device 30 includes the winding body 32 composed of the humidifying tube 31 that supplies moisture present outside to the high-concentration oxygen flowing through the interior, the winding body 32 being configured by winding the single humidifying tube 31 around the virtual axis P.
   The humidifying device 30 of the oxygen concentrator M of the above embodiment includes the winding body 32 configured by winding the humidifying tube 31. The winding body 32 of the humidifying tube 31 has less surface overlap between the humidifying tubes 31, as compared to the case where the humidifying tubes 31 are used in a bundle. Therefore, the humidifying device 30 has a larger surface area of the humidifying tube 31 that can be effectively utilized for acquiring moisture, as compared to the case where the same total length of the humidifying tube 31 is used in a bundle. Therefore, the humidifying device 30 with such a configuration can enhance the humidification capacity for the high-concentration oxygen, as compared to conventional humidifying devices. This allows the oxygen concentrator M including the humidifying device 30 of the type that uses the humidifying tube 31 to increase the amount of humidification for the high-concentration oxygen, as compared to conventional oxygen concentrators.
(2) In the oxygen concentrator M of the above embodiment, the winding body 32 has a form that is wound in a spiral shape around the virtual axis P.
   The humidifying device 30 including the winding body 32 with such a configuration can effectively utilize the surface area of the humidifying tube 31 for acquiring moisture. This allows the humidification capacity of the humidifying device 30 for the high-concentration oxygen to be enhanced.
(3) In the oxygen concentrator M of the above embodiment, the winding body 32 has a form that is wound in a helical shape around the virtual axis P.
   The humidifying device 30 including the winding body 32 with such a configuration can effectively utilize the surface area of the humidifying tube 31 for acquiring moisture. This allows the humidification capacity of the humidifying device 30 for the high-concentration oxygen to be enhanced.
(4) In the oxygen concentrator M of the above embodiment, the humidifying device 30 further includes the holder 35 that holds the winding body 32. The holder 35 holds the humidifying tubes 31 in a state in which the adjacent humidifying tubes 31 are spaced apart from each other.
   The humidifying device 30 including the holder 35 with such a configuration can make maximum effective use of the surface area of the humidifying tube 31 for acquiring moisture. This allows the humidification capacity of the humidifying device 30 for the high-concentration oxygen to be further enhanced.
(5) In the humidifying device 30 of the above embodiment, the winding body 32 includes the first winding body 32a and the second winding body 32b. The first winding body 32a and the second winding body 32b are disposed coaxially adjacent to each other in the direction of the virtual axis P.
   The oxygen concentrator M including the winding body 32 with such a configuration, which includes the two humidifying tubes 31, can increase the surface area of the humidifying tubes 31 that can be utilized for acquiring moisture in the humidifying device 30.
(6) The oxygen concentrator M of the above embodiment further includes the air pump 23 that supplies raw air to the adsorption cylinder 20, the cooling fan 26 that blows cooling air to the air pump 23, the air supply ports 13 and 15 that take in cooling air from the outside, and the casing 10 and the fan box 12 that constitute the flow path of the cooling air flowing from the air supply ports 13 and 15 to the cooling fan 26. In the oxygen concentrator M of the above embodiment, the humidifying device 30 is disposed at a position away from the flow F1 of the cooling air in the flow path.
   The oxygen concentrator M with such a configuration can enhance the flexibility of the placement of the humidifying device 30 by disposing the humidifying device 30 at a position away from the flow F1 of the cooling air generated by the cooling fan 26. This allows the space efficiency of the oxygen concentrator M including the humidifying device 30 to be enhanced, enabling further downsizing of the oxygen concentrator M.
(7) In the oxygen concentrator M of the above embodiment, the humidifying device 30 is disposed outside the fan box 12.

The oxygen concentrator M with such a configuration can further enhance the flexibility of the placement of the humidifying device 30 by disposing the humidifying device 30 outside the fan box 12. This allows the space efficiency of the oxygen concentrator M including the humidifying device 30 to be enhanced, enabling further downsizing of the oxygen concentrator M.

While various embodiments have been described herein above, it is to be appreciated that various changes in form and detail may be made without departing from the spirit and scope presently or hereafter claimed.

### REFERENCE SIGNS LIST

- M: oxygen concentrator
- 10: casing (housing)
- 12: fan box (cooling fan housing)
- 13: air supply port
- 15: air supply port
- 20: adsorption cylinder
- 23: air pump
- 26: cooling fan
- 30: humidifying device
- 31: humidifying tube
- 32: winding body
- 32a: first winding body (first winding body)
- 32b: second winding body (second winding body)
- 35: holder
- F1: flow (of cooling air)
- P: virtual axis

## Claims

1. An oxygen concentrator (M) comprising: an adsorption cylinder (20) having an adsorbent that selectively adsorbs nitrogen from raw air and generates high-concentration oxygen; and a humidifying device (30) that humidifies the generated high-concentration oxygen,
wherein the humidifying device (30) includes a winding body (32) composed of a humidifying tube (31) that supplies moisture present outside to the high-concentration oxygen flowing through an interior, the winding body (32) being formed by winding the single humidifying tube (31) around a virtual axis (P).

2. The oxygen concentrator (M) according to claim 1, wherein the winding body (32) has a form that is wound in a spiral shape around the virtual axis (P).

3. The oxygen concentrator (M) according to claim 1 or 2, wherein the winding body (32) has a form that is wound in a helical shape around the virtual axis (P).

4. The oxygen concentrator (M) according to claim 1 or 2, wherein
the humidifying device (30) further includes a holder (35) that holds the winding body (32), and
the holder (35) holds the humidifying tube (31) in a state where the adjacent humidifying tubes (31) are spaced apart from each other.

5. The oxygen concentrator (M) according to claim 1 or 2, wherein
the winding body (32) includes a first winding body (32a) and a second winding body (32b), and
the first winding body (32a) and the second winding body (32b) are disposed coaxially adjacent to each other in a direction of the virtual axis (P).

6. The oxygen concentrator (M) according to claim 1 or 2, further comprising:
an air pump (23) that supplies the raw air to the adsorption cylinder (20);
a cooling fan (26) that blows cooling air to the air pump (23); and
a housing (10, 12) that includes an air supply port (13, 15) that takes in the cooling air from outside and constitutes a flow path for the cooling air that flows from the air supply port (13, 15) to the cooling fan (26),
wherein the humidifying device (30) is disposed at a position away from a flow (F1) of the cooling air in the flow path.

7. The oxygen concentrator (M) according to claim 6, wherein
the housing includes a cooling fan housing (12) that houses the cooling fan (26), and
the humidifying device (30) is disposed outside the cooling fan housing (12).
